(19) **Europäisches Patentamt European Patent Office Office européen des brevets**

(11) **EP 1 777 299 A1**

(12) **EUROPEAN PATENT APPLICATION**

(43) Date of publication:
**25.04.2007 Bulletin 2007/17**

(51) Int Cl.:
**C12Q 1/68** (2006.01)

(21) Application number: **06021286.7**

(22) Date of filing: **11.10.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **11.10.2005 EP 05022084**

(71) Applicants:
• **Roche Diagnostics GmbH**
  **68305 Mannheim (DE)**
  Designated Contracting States:
  **DE**
• **F. HOFFMANN-LA ROCHE AG**
  **4070 Basel (CH)**
  Designated Contracting States:
  **AT BE BG CH CY CZ DK EE ES FI FR GB GR HU IE IS IT LI LT LU LV MC NL PL PT RO SE SI SK TR**

(72) Inventors:
• **Fuerst, Roderic**
  **82377 Penzberg (DE)**
• **Kopal, Guido**
  **82377 Penzberg (DE)**

(54) **Combination of optical mapping with ordered restriction maps**

(57)    The present invention is directed to a method for de novo assembly of genomic sequence information comprising the combination optical whole genome restriction mapping an ultra high throughput pyrosequencing.

EP 1 777 299 A1

**Description**

The present invention relates to the problem of aligning sequence information derived from a shotgun sequencing approach. More precisely, the present invention provides a new algorithm for combining data obtained from an ordered restriction map with data obtained from a shotgun sequencing by synthesis procedure.

Technical prior art background

Typically the DNA of interest is cloned into a plasmid vector. Subsequently, a sample of this plasmid is digested with a set of individual enzymes. The length of fragments of DNA generated in the aforementiond digestion process are characterized using agarose gel electrophoresis. From the lengths of the fragments the location of the restriction endonuclease cutting sites can be deduced.

In the case of optical restriction mapping, individual molecules of DNA are digested when they are immobilized to a solid phase and the sizes of the resulting fragments are directly measured by the analysis of optical images.

Whole genome DNA Sequencing technology has become an important tool for biomedical research even after sequencing of the humane genome has been completed. Sequence information from individual specimens of bacterial organisms for example is used for the identification of particular strains, within population studies, or the de novo generation of antibiotic resistencies. Sequence information obtained from human individuals is used for example, to study polymorphisms and their associaciton with complex inherited or pathogenic predispositions.

Besides the well known methods of sequencing such as Sanger-Di-deoxy-sequencing and Maxam-Gilbert-sequencing, there is a third sequencing principle known in the art which is gernerally refered to as sequencing by synthesis. This principle is based on a primer extension reaction catalyzed by a DNA polymerase in the presence of one defined modified or unmodified nucleoside triphosphate and subsequent direct or indirect detection of the generated chemical side product derived from said primer extension reaction. In one particular embodiment, generation of Pyrophosphate is detected indirectly (US 4,863,849, WO 92/16654, US 6,210,891, US 6,258,568) (Ronaghi, M., et al., Analytical Biochemistry 242 (1996) 84-89) (Ronaghi, M., et al., Science 281 (1998) 363-365).

Recently, an ultra-high throughput sequencing system based on Pyrophosphate sequencing was disclosed which allows for the sequencing of a bacterial genome in essentially not more than one week (WO 04/70007, WO 05/03375). Starting from sheared genomic DNA, single fragments are bound to beads which are captured in a PCR-reaction-mixture-in-oil emulsion (WO 04/69849). Amplification then results in a library of clonally amplified DNA with each bead carrying multiple copies of the same fragment. After breakage of the emulsion and denaturation of the PCR products into single strands, beads are deposited into the multiple wells of a fiber-optic picotiter plate such that one well carries not more than a single bead. More than 1 000 000 pyrophosphate sequencing reactions are then carried out simultaneously. The generation of Pyrophosphate is triggering a luminescent reaction cascade and light is finally detected with a CCD camera.

The Bio-Informatics of such a Genome sequencing system allow for both the confirmation sequencing approach and the de-novo sequencing approach. In the confirmation sequencing approach, the sequence information obtained is aligned to an already known sequence and differences such as SNPs are identified. In the de novo sequencing approach, the sequence information obtained from single reads is analyzed for overlaps between each of the reads and so called contigs of consitutive sequence information are built as far as possible. This may help for example in order to identify a specific bacterial strain or even a mixture of different microorganisms.

Brief description of the invention

The present invention is directed to a method for de novo assembly of genomic sequence information, comprising the steps of

(i) using genomic DNA obtained from a specific organism for a method to generate sequence information by means of

- subjecting said genomic DNA to a procedure of clonally isolating and amplifying a library of single stranded DNA molecules.
- subjecting said clonally amplified and isolated library to a sequencing by synthesis reaction, in order to create whole genome shotgun sequence information
- obtaining sequence reads and assembling contigs composed thereof;

(ii) using genomic DNA obtained from the same organism for whole genome optical restriction mapping with at least one restriction enzyme in order to generate an ordered restriction map;
(iii) aligning the sequence information obtained from steps (i) and (ii) such that the sequence contigs are orientated and ordered with respect to the ordered restriction map obtained in step (ii).

In some cases it is advantageous if the genomic DNA is isolated once and is subsequently size fractionated and fragments of a smaller size are taken to generate sequence information according to step (i), whereas fragments of a larger size are taken to generate an ordered restriction map according to step (ii).

In one embodiment the method according to the present invention further comprises the steps of

- identification of at least one sequence gap which is not covered by a contig, and
- length determination of said sequence gap.

It is also within the scope of the present invention, if based on the sequence information obtained, the following steps are performed:

- identification of appropriate primer sequences capable of amplifying a DNA fragment covering a sequence gap
- performance of a PCR reaction with a mixture comprising a Taq DNA polymerase and a thermostable DNA polymerase with proofreading activity in order to amplify said DNA fragment
- sequencing said DNA fragment.

In another embodiment, the contigs obtained from step (i) are subjected to a validity test based on the ordered restriction map obtained in step (ii).

Preferably, after contigs which have failed to pass the validity test have been identified, the sequence reads obtained in step (i) are reassembled without allowing recreation of those contigs which have failed to pass said validity test.

Detailed Description of the invention

In general, the present invention is directed to a method for the de novo assembly of genomic sequence information, comprising the steps of

- using genomic DNA obtained from a specific organism for a method to generate sequence information by means of

    (i) subjecting said genomic DNA to a procedure of clonally isolating and amplifying a library of single stranded DNA molecules
    (ii) subjecting said clonally amplified and isolated library to a sequencing by - synthesis reaction, in order to create whole genome shotgun sequence information;
    (iii) obtaining sequence reads and assembling contigs from said sequence reads as sequence information;

- using genomic DNA obtained from the same organism for whole genome ordered restriction mapping with at least one restriction enzyme in order to generate an ordered restriction map;
- aligning the sequence information obtained from steps (ii) and (iii) such that the sequence contigs are orientated and ordered with respect to the ordered restriction map obtained in step (ii).

In the context of the present invention, the following definitions shall apply:

"Sequence information" shall mean the order of nucleotide residues of at least a part of a genome.

"Aligning sequence information" shall mean comparing different sequence informations with each other and identifying regions of identity or overlap.

"De novo assembly of genomic sequence information" shall mean assembly of sequence information by repeatedly comparing the sequences obtainend from sequence reads or contigs with each other without using sequence information from an external source.

"Whole genome shotgun sequence information" shall mean the plurality of sequence information obtained from a de novo assembly of genomic sequence information, characterized in that sequence information was obtained from arbitrarily generated sequence reads.

"Clonal isolation and amplification of a library" shall mean that all members of a genomic library are physically separated from each other and subsequently amplified.

"Sequencing by synthesis" shall mean that a primer extension reaction is performed, where the 4 different A,G, C, and T nucleoside triphosphates or their respective analogs are supplied in a repetitive series of events and the sequence of the nascent strand is infered from chemical products derived from the extension reaction catalyzed by the DNA polymerase. In a particular embodiment, the sequencing by synthesis method is a pyrophosphate sequencing method, characterized in that generation of pyrophosphate is detected as follows:

$$PPi + adenosine\ 5'\ phosphosulfate\ (APS) \rightarrow ATP,\ catalyzed\ in\ the\ presence\ of\ Apyrase$$

$$ATP + luciferin \rightarrow light + oxy\ luciferin,\ catalyzed\ in\ the\ presence\ of\ Luciferase.$$

Luminescence of oxy luciferin can then become detected by a CCD camera.

"Sequence read" shall mean the sequence information obtained in one sequencing by synthesis reaction.

"Contig" shall mean the sequence information relating to a contigous series of bases infered from a number of sequence reads which overlap to such an extend that an overall alignment is possible.

"Ordered restriction mapping" shall mean providing information on at least a part of a genome with respect to the number and lengths of its restriction fragments and on the order of said fragments as they occur within said genome.

"Whole genome ordered restriction map" shall mean a restriction map of a complete genome.

"Optical restriction mapping" shall mean a method of ordered restriction mapping, characterized in that information on the order of restriction fragments is obtained by optical means.

Purification and isolation of genomic DNA for the mapping procedure needs to be done smoothly and with great care in order to avoid undesired shearing events as far as possible. For example, genomic DNA can be prepared according to Zhou, S., et al., Mol. Biochem. Parasitol. 138 (2004) 97-106 (2004).

It is also within the scope of the present invention, if isolated genomic DNA is originating from a source comprising different viruses or different microorgansisms. Examples for such samples are samples harvested from feces, intestine ort he respiratory tract of a mammalian or in particular human individuum.

In case the source of genomic DNA is basically available without limitation, as it is the case for DNA obtainable from a cultivated microorganism or a eucaryotic tissue culture, it is thus advantageous to isolate genomic DNA for the mapping and the sequencing procedures separately.

However, if the source of DNA is limited, it may be advantageous to isolate the genomic DNA once and prepare at least two aliquots, one of which is used for the mapping procedure and the second of which is used for the sequencing procedure. In a specific embodiment, the obtained genomic DNA is size fractionated by conventional methods known in the art. In this context, prefered methods are based on chromatographic (Kasai, K., Journal of Chromatography 618 (1993) 203-221), or electrophoretic methods (Dear, J., et. al., Biochemical Journal 273 (1991) 695-699).

Fragments of a smaller size are taken to generate sequence information according to step (i), whereas fragments of a larger size are taken to generate an ordered restriction map according to step (ii). Preferably a third aliquot is stored and used at a later time point in order to perform additional sequencing reactions specifically designed to fill gaps of the assembled overall sequence.

One important step of the method according to the present invention is the step of subjecting said genomic DNA to a procedure of clonally isolating and amplifying a library of single stranded DNA molecules as disclosed in WO 04/70007.

In a first step, the genomic DNA is randomly fragmented by any method known in the art, but preferably by means of nebulization (WO 92/07091). In a second step, specifically designed adaptors are ligated to the ends of the genomic fragments. In a third step, individual fragments are captured via the adaptors onto their own beads. In a fourth step, said beads together with amplification reagents are mixed with an appropriate oil to prepare an emulsion and within each hydrophilc droplet, a clonal amplification by means of PCR takes place. Newly synthesized strands remain within their droplets and are bound to the respective bead.

Another important feature of the method according to the present invention is the step of subjecting said clonally amplified and isolated library to a sequencing by - synthesis reaction, in order to create whole genome shotgun sequence information as disclosed in WO 05/03375. In a first step, the emulsion is broken preferably by means of filtering said emulsion and subsequent harvesting of the beads carrying the clonally amplified library. The beads are then deposited into wells of a fiber optic slide, which can be a picotiter plate. The sizes of the beads and the wells of the picotiter plate are adjusted to each other in such a way that only one bead per well can be deposited. The picotiter plate is then inserted into a flow chamber and the base of the slide is in optical contact with fiber-optic bundle connected to a CCD camera, allowing capture of photons from each individual well. Pyrophosphate sequencing is then performed by using Apyrase- and Luciferase-coupled beads for the generation of detectable photons. These beads are much smaller than the beads comprising the amplified DNA so that multiple enzyme coupled beads fit in each well of the microtiter plate. For the reaction itself, reagent mixtures including Bst Polymerase and A, G, C, or T Nucleoside triphosphates subsequently one after another are cyclically delivered through the flow system. Depending on the template sequence, primer extension eventually occurs.

Details of the methods for clonal amplification of a random genomic library and high throughput sequencing by synthesis are also found in Margulies, M., et al., Nature 437 (2005) 376-80.

At first, all pairwise overlaps between fragments are identified by comparison of the flow signals of all possible read pairs. The dot product of the normalized flow signals of the fragments is calculated and fragments with a dot product above a certain threshold are used to assemble larger contiguous unique sequences ("unitigs"). Unitigs are built from a sequence of maximum depth overlapping reads. A unitig ends where a repeat region or completely unsequenced region starts. All read signals are aligned and the average flow signal at a specific position is calculated and used for the consensus base call of the unitigs. After the final consensus base call, three optimization steps are carried out. First, an all_against_all unitig comparison is carried out and overlapping unitigs are joined. In the second optimization step, reads which span the end of 2 unitigs are used to join them. In both steps repeat region boundaries are identified to avoid a join of contigs containing these repeat boundaries. Finally all reads used to build unitigs are mapped against the consensus sequence. Contigs with a region of less than 4 spanning reads are broken and only contigs larger than 500 bp are kept for output.

A still other important feature of the method according to the present invention is the step of ordered restriction mapping, i.e. providing a restriction map preferably of a whole genome.

In a particular embodiment, the ordered restriction map is generated by the process of optical restriction mapping. A fluid flow is used to stretch out DNA molecules dissolved in molten agarose and fix them in place during gelation. The gelation process restrains elongated molecules from relaxing to a random coil conformation during enzymatic cleavage. A restriction enzyme is added to the molten agarose-DNA mixture, and cutting is triggered by the diffusion of Mg2 plus into the gelled mixture, which has been mounted on a microscope slide. Fluorescence microscopy coupled with digital image processing techniques is used to record, at regular intervals, cleavage sites, which are visualized by the appearance of growing gaps in imaged molecules and bright, condensed pools or "balls" of DNA on the fragment ends flanking the cut site. These balls form shortly after cleavage as a result of coil relaxation at the new ends. The size of the resulting fragments is determined in two ways: by measurement of the relative fluorescence intensities of the products and by measurement of the relative apparent DNA molecular lengths in the fixating gel. Maps are subsequently assembled by recording the order of the sized fragments. Averaging a small number of molecules rather than using only one improves accuracy and permits rejection of unwanted molecules (Schwartz, D.C., et al., Science 262 (1993) 110-114).

The step of aligning the sequence information such that the sequence contigs are orientated and ordered with respect to the ordered restriction map obtained in step (ii) reveals information on the location and size of gap regions, for which no further sequence information from a contig is available so far.

Therefore, in one major aspect, the present invention is directed to a method further comprising the steps of

- identification of at least one sequence gap which is not covered by a contig, and
- length determination of said sequence gap,
- identification of appropriate binding sites for amplification primers suitable to amplify a DNA fragment comprising said gap.

Depending on the size of the sequence gap, there are different preferred possibilities within the scope of the present invention in order to obtain sequence information from the gap regions.

a) Small gaps < 0.5 kb

For small gaps, amplification primers may be designed from the sequence information of adjacent contigs for a conventional PCR amplification reaction. Subsequently, the amplification product may become sequenced directly by the dideoxy method using primers, the sequences of which can be deduced from known sequences from both sides which are flanking the gap. In a particular embodiment, at least one or two sequencing primers is/are identical to all or at least a part of the amplification primers that have been used.

b) Medium sized gaps < 10 kb

Also for this type of gaps, primers may be designed form the sequence information of adjacent contigs for a PCR amplification reaction. Yet, in order to obtain such long PCR fragments, it is highly desirable to use an appropriate amplification reagent mix with a high degree of processivitiy and simultaneously a high degree of accuracy. Thus, preferably such a reaction mixture comprises a mixture of polymerases comprising a Taq DNA polymerase and a thermostable DNA polymerase with proofreading activity in order to amplify the desired DNA fragment representing the sequence gap. The amplified fragments may be sequenced by any method known in the art, preferably a di-deoxy-sequencing method, using primers, the sequences of which can be deduced from known sequences from both sides which are flanking the gap. In a particular embodiment, at least one or two sequencing primers is/are identical to all or at least a part of the amplification primers that have been used. Furthermore, additional sequence information can be obtained by a conventional primer walking approach.

c) Large gaps >10 kb

For these types of gaps, it is within the scope of the present invention if based on the sequence information available from the terminal regions of the two contigs adjacent to the gap, hybridization probes are designed which can be used in order to screen genome libraries like libraries cloned into a plasmid or cosmid vector or a Yeast artificial chromosomes according to methods which are well known in the art. In case of libraries with very large inserts, it is sufficient in most cases to further sequence those clones, which have been identified to be detected by both hybridization probes each derived from one terminal sequence of the two respective adjacent contigs.

In a second major embodiment, the present invention is applicable for validating the sequence data obtained from the sequencing by synthesis reaction using the results obtained from the ordered mapping procedure and vice versa.

Thus, the present invention is also directed to a method of generating mapping and sequence data and subsequently aligning those two classes of data as disclosed above, further characterized in that the contigs obtained from step (i)are subjected to a validity test based on the ordered restriction map obtained in step (ii).

Without limiting the scope of the present invention, such a validation algorithm may be as follows:

In a first step, the pool of generated contigs is separated into two categories. The first category contains all sequence contigs, whose length and sequence is not in contradiction with any of the restriction sites which have been identified by the mapping procedure. The information of these contigs does not need to become processed any further.

The second category of contigs contains all contigs characterized in that their length and/or sequence is indeed in contradiction with any of the restriction sites which have been identified by the mapping procedure. Yet, there needs to be established a tolerance interval with respect to the length parameter, since length measurement for ordered mapping in some cases turns out to be not absolutely accurate.

The reason underlying the contradiction can either be wrong mapping data or altenratively, wrong contig information.

Wrong sequencing data in most cases are due either to

- misinterpretation of primary signal detection, or
- a wrong fusion of sequence reads or a plurality of sequence reads, or
- repetitive sequences.

Improvement of sequence information can for example be corrected by the following 3 algorithms which can be applied either each alone or subsequently in the order indicated below.

a) In one embodiment, improvement of the contig sequence information is obtained by means of

- identifying sequences within the contigs which differ from a RE recognition sequence
- comparing those sequences with respect to the data obtained from the mapping procedure,
- if the mapping data reveal a putative RE recognition sequence at the respective site, correcting the contig sequence to include the respective site.

b) If the mapping data reveal a particular restriction site at a sequence within a contig which is not represented in the sequence information of the contig as available, the contig may be an artificial contig that has been generated due to a false fusion of partial sequence information available. In this case, starting from the contig as defined originally, sub-contigs may be defined, which are in complete accordance with the information obtained from the mapping procedure. Those sub-contigs are then further regarded as validated contigs and become members of the first contig category.

c) If the mapping data reveal a fragment length which does not correspond to the sequence information of a contig containing repetitive sequences, due to the nature of sequencing by synthesis it is often the case that the assembled contig sequence is too short and the mapping data are more reliable. Thus, in cases, where a contig sequence reveals a repetitive sequence, which is either a mononucleotide repeat, a dinucleotide repeat or a trinucleotide repeat or a polynucleotide repeat or even a partial or complete gene duplication, the contig sequence needs to be corrected by an alternative re-sequencing approach.

As already indicatd above, improvement of the contig sequence information in some cases may be hampered by the generation of wrong mapping information. Wrong mapping data are predominantly due either to

- a repeated failure to cut the genomic DNA at a certain position, or, alternatively
- a repeated systematic error in appropriate fragment length measurement.

In oder to eliminate these mistakes, prior to subjecting the contig sequence data to validation by means of comparing them with the mapping data, the information obtained from the mapping data may be improved by information available from the contigs. Improvement of sequence information can for example be corrected by the following 3 algorithms which can be applied either each alone or one after another.

a) In one particular embodiment, the length of a single or several restriction fragments identified by the contig sequence information, for which the corresponding restriction fragment identified by ordered optical mapping has been identified, can be used to calibrate the length of all restriction fragments identified by the ordered optical restriction mapping.

b) In case a certain restriction site has not been identified by the ordered mapping procedure thus resulting in longer fragment information, there are two possibilities. First, a consensus basecall for each nucleotide in a contig can be defined. In case the average consensus base call of all positions of a restriction site identified by sequencing exceeds a certain predefined cut off value, the information on the position of this restriction site is included into the data set of the mapping result. Alternatively, a basecall obtained by sequencing can be defined as being sufficient to provide a basis for an amendment of the mapping information, in case the respective position has been sequenced in depth thereby providing a high level of confidence, i.e. a certain predefined number of sequence reads covering this position have been performed.

c) Frequently, fragments under a certain length of about 200 base pairs are not identified by the optical mapping procedure. In case such small restriction fragments under a predefined cut off length are present in contigs which have been deduced from the sequencing procedure, the information on said fragments can be added to the overall ordered mapping information obtained from the optical mapping procedure.

Starting from the corrected mapping information, improvement of the contig sequence information can be obtained by any algorithm as disclosed above or any combination thereof. Thus, the present invention is directed to a computer program product comprising a software to compare and/or align a whole genome ordered restriction map with multiple contigs obtained from a sequencing by synthesis reaction. Such a software program is able to associate the content of a database comprising infromation on contigs with the content of a database comprising information on an ordered restriction map. In addition, the present invention is directed to a respective computer-readable medium or a computer-readable storage medium comprising such a computer program product.

The following example is provided to aid the understanding of the present invention, the true scope of which is set forth in the appended claims. It is understood that modifications can be made in the procedures set forth without departing from the spirit of the invention.

Example:

Genomic DNA derived from a single clone of a bacterial isolate is purified using the MagNa Pure Instrument (Roche Diagnostics Cat. No. 12 236 931 001) according to instructions of the distributor using the MagNa Pure LC DNA Isolation kit III (Roche Diagnostics Cat. No. 03 264 785 001). One aliquot of the isolated genomic DNA is then subjected to a method of optical mapping as disclosed in Zhou, S., et al., Genome Research 13 (2003) 2142-2151, using the restriction enzymes Eco RI and Hind III in order to obtain an ordered restriction map. A second aliquot is subjected to a large scale sequencing by synthesis process and a de novo shotgun sequence assembler as disclosed in Margulies, M., et al., Nature 437 (2005) 376-80. The obtained sequence infromation is used to identify the bacterial species of the isolate. The data are confirmed by the information obtained from the optical restriction map.

**Claims**

1.  A method for de novo assembly of genomic sequence information, comprising the steps of

    (i) using genomic DNA obtained from a specific organism for a method to generate sequence information by means of

    - subjecting said genomicDNA to a procedure of clonally isolating and amplifying a library of single stranded DNA molecules
    - subjecting said clonally amplified and isolated library to a sequencing by synthesis reaction, in order to create whole genome shotgun sequence information
    - obtaining sequence reads and assembling contigs composed thereof

    (ii) using genomic DNA obtained from the same organism for whole genome optical restriction mapping with at least one restriction enzyme in order to generate an ordered restriction map
    (ii) aligning the sequence information obtained from steps (i) and (ii) such that the sequence contigs are orientated and ordered with respect to the ordered restriction map obtained in step (ii).

2.  A method according to claim 1, further **characterized in that** the genomic DNA is isolated once and is subsequently size fractionated and fragments of a smaller size are taken to generate sequence information according to step (i), whereas fragments of a larger size are taken to generate an ordered restriction map according to step (ii).

3.  A method according to claim 1, further comprising the steps of

    - identification of at least one sequence gap which is not covered by a contig
    - length determination of said sequence gap.

4.  A method according to claim 3, further comprising the steps of

    - identifying appropriate primer sequences capable of ampigfying a DNA fragment covering said sequence gap
    - performing a PCR reaction with a mixture comprising a Taq DNA polymerase and a thermostable DNA polymerase with proofreading activity in order to amplify said DNA fragment
    - sequencing said DNA fragment.

5.  A method according to claim 1, further **characterized in that** the contigs obtained from step (i) are subjected to a validity test based on the ordered restriction map obtained in step (ii).

6.  A method according to claim 5 further comprising

    - identifying a contig which has failed to pass the validity test
    - reassembling the sequence reads obtained in step (i) without allowing recreation of said contigs which have failed to pass said validity test.

7.  Computer program product comprising a software to compare and/or align a whole genome ordered restriction map with multiple contigs obtained from a sequencing by synthesis reaction.

**European Patent Office**

# EUROPEAN SEARCH REPORT

Application Number

EP 06 02 1286

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ZHOU SHIGUO ET AL: "Whole-genome shotgun optical mapping of Rhodobacter sphaeroides strain 2.4.1 and its use for whole-genome shotgun sequence assembly." September 2003 (2003-09), GENOME RESEARCH, VOL. 13, NR. 9, PAGE(S) 2142-2151 , XP002354221 ISSN: 1088-9051 * page 2142, column 2, paragraph 1 * * page 2143, column 2, paragraph 2 * * page 2143, column 2, paragraph 3 * * page 2144, column 1, paragraph 2 * * page 2147, column 1, paragraphs 2,3 * * figure 3 * | 1-7 | INV. C12Q1/68 |
| X,D | ZHOU S ET AL: "Shotgun optical mapping of the entire Leishmania major Friedlin genome" MOLECULAR AND BIOCHEMICAL PARASITOLOGY, ELSEVIER SCIENCE PUBLISHERS, AMSTERDAM, NL, vol. 138, no. 1, November 2004 (2004-11), pages 97-106, XP004611806 ISSN: 0166-6851 * the whole document * | 1-7 | TECHNICAL FIELDS SEARCHED (IPC) C12Q |
| X | ZHOU SHIGUO ET AL: "A whole-genome shotgun optical map of Yersinia pestis strain KIM." December 2002 (2002-12), APPLIED AND ENVIRONMENTAL MICROBIOLOGY, VOL. 68, NR. 12, PAGE(S) 6321-6331 , XP002354222 ISSN: 0099-2240 * the whole document * | 1-7 | |

-/--

The present search report has been drawn up for all claims

| Place of search | Date of completion of the search | Examiner |
|---|---|---|
| Munich | 22 November 2006 | Helliot, Bertrand |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

**EUROPEAN SEARCH REPORT**

Application Number

EP 06 02 1286

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LIM ALEX ET AL: "Shotgun optical maps of the whole Escherichia coli O157: H7 genome" September 2001 (2001-09), GENOME RESEARCH, VOL. 11, NR. 9, PAGE(S) 1584-1593 , XP002354223 ISSN: 1088-9051 * the whole document * | 1-7 | |
| A | WO 2004/046889 A (NEW YORK UNIVERSITY; MISHRA, BUD; ANANTHARAMAN, THOMAS) 3 June 2004 (2004-06-03) | 1-7 | |

TECHNICAL FIELDS
SEARCHED      (IPC)

The present search report has been drawn up for all claims

| Place of search | Date of completion of the search | Examiner |
|---|---|---|
| Munich | 22 November 2006 | Helliot, Bertrand |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 ...............................................................................

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 06 02 1286

This annex lists the patent family members relating to the patent documents cited in the above-mentioned European search report.
The members are as contained in the European Patent Office EDP file on
The European Patent Office is in no way liable for these particulars which are merely given for the purpose of information.

22-11-2006

| Patent document cited in search report | Publication date | Patent family member(s) | Publication date |
|---|---|---|---|
| WO 2004046889 A | 03-06-2004 | AU 2003294391 A1 | 15-06-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4863849 A
- WO 9216654 A
- US 6210891 B
- US 6258568 B

- WO 0470007 A
- WO 0503375 A
- WO 0469849 A
- WO 9207091 A

**Non-patent literature cited in the description**

- **RONAGHI, M. et al.** *Analytical Biochemistry,* 1996, vol. 242, 84-89
- **RONAGHI, M. et al.** *Science,* 1998, vol. 281, 363-365
- **ZHOU, S. et al.** *Mol. Biochem. Parasitol.,* 2004, vol. 138, 97-106
- **KASAI, K.** *Journal of Chromatography,* 1993, vol. 618, 203-221

- **DEAR, J.** *Biochemical Journal,* 1991, vol. 273, 695-699
- **MARGULIES, M. et al.** *Nature,* 2005, vol. 437, 376-80
- **SCHWARTZ, D.C. et al.** *Science,* 1993, vol. 262, 110-114
- **ZHOU, S. et al.** *Genome Research,* 2003, vol. 13, 2142-2151